# EUROPÄISCHE PATENTSCHRIFT

(11) **EP 1 961 400 B1**
(45) Veröffentlichungstag und Bekanntmachung des Hinweises auf die Patenterteilung: **17.08.2011**
(21) Anmeldenummer: 07033543.5
(22) Anmeldetag: 07.11.2007
(51) Int. Cl.: A61F 2/18

(54) **Gehörknöchelchenprothese**
Auditory ossicle prosthesis
Prothèse d'osselet

(30) Priorität: 23.02.2007 DE 102007008851
(43) Veröffentlichungstag der Anmeldung: 27.08.2008
(73) Patentinhaber: Heinz Kurz GmbH Medizintechnik, 72144 Dusslingen (DE)
(72) Erfinder: Steinhardt, Uwe, 72145 Hirrlingen (DE); Huber, Alex, PD Dr. med., 8700 Küsnacht (CH); Eiber, Albrecht, Dr.-Ing., 71384 Weinstadt (DE)
(74) Vertreter: Kohler Schmid Möbus

(56) Entgegenhaltungen:
- EP-A- 1 073 313
- DE-A1-102004 038 078
- DE-U1- 29 609 687
- US-A1- 2001 037 151

## Beschreibung

Die Erfindung betrifft eine passive Gehörknöchelchenprothese, die mindestens ein Glied der menschlichen Gehörknöchelchenkette ersetzt oder überbrückt, wobei die Gehörknöchelchenprothese zumindest an einem Ende ein erstes Befestigungselement zur mechanischen Verbindung mit einem Glied der Gehörknöchelchenkette, nämlich mit dem Ambossfortsatz oder mit dem Hammergriff, aufweist, welches in Form einer das Glied umgebenden, durch eine spaltartige Öffnung nach außen hin teilweise offenen Schlinge gestaltet ist, die das Glied der Gehörknöchelchenkette, mit dem die mechanische Verbindung hergestellt werden soll, kraftschlüssig umgreift, und wobei die spaltartige Öffnung der Schlinge erheblich kleiner ist als der Durchmesser des von der Schlinge umgriffenen Gliedes.

Eine solche passive Gehörknöchelchenprothese ist beispielsweise aus dem deutschen Gebrauchsmuster DE 296 09 687 U1, aus der WO 02/069850 A1 oder aus der US 6,554,861 B2 bekannt.

Das menschliche Mittelohr mit seinen Gehörknöchelchen hat die Aufgabe, die über den äußeren Gehörgang auf das Trommelfell auftreffenden Schallwellen auf das mit Flüssigkeit gefüllte Innenohr zu übertragen. Die drei Gehörknöchelchen sind der am Trommelfell befestigte Hammer (lat. Malleus), der Steigbügel (lat. Stapes), der über seine Fußplatte (lat. Basis stapedis) mit dem Innenohr verbunden ist, und der Amboss (lat. Incus), der sich zwischen dem Hammer und dem Steigbügel befindet und mit diesen gelenkig verbunden ist. Bei der Otosklerose, einer Erkrankung des menschlichen Felsenbeins (=Knochen, in dem das gesamte Ohr sitzt), kann es durch entzündungsähnlichen Knochenumbauprozesse zu einer Fixierung des normalerweise locker schwingenden Steigbügels kommen. Dadurch wird das Schallsignal nicht oder nur unvollkommen über die Gehörknöchelchenkette auf das Innenohr übertragen, was zur Schwerhörigkeit führt.

Passive Gehörknöchelchenprothesen werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den individuellen Gegebenheiten beim Patienten das eine Ende der Gehörknöchelchenprothese beispielsweise am Ambossfortsatz oder am Hammergriff fixiert und das andere Ende beispielsweise am Steigbügel befestigt oder direkt ins Innenohr getaucht wird. Vielfach wird mit den bekannten Gehörknöchelchenprothesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Ein Hauptproblem beim Einsetzen einer Gehörknöchelchenprothese liegt in der Fixierung der Prothese an einem Glied der Gehörknöchelchenkette, die einerseits natürlich sicher und dauerhaft sein soll, andererseits aber auch vom Operateur auf halbwegs einfache Weise zu bewerkstelligen sein muss. Beispielsweise aus der US 3,711,869 A oder aus der US 5,370,689 A sind Gehörknöchelchenprothesen bekannt, die mittels einer geschlitzten Öse am Ambossfortsatz befestigt werden. Zum Aufweiten der Öse und zur Erleichterung des Aufschiebens der Prothese auf den Ambossfortsatz sind jedoch Spezialwerkzeuge erforderlich, ebenso für die Fixierung der Prothese durch Zusammendrücken der geschlitzten Öse, was sich in der Praxis als vergleichsweise umständlich erweist.

In der DE 10 2004 038 078 A1 sind implantierbare aktive Hörgeräte beschrieben, von denen Teile - ähnlich wie bei einer passiven Gehörknöchelchenprothese - Glieder der menschlichen Gehörknöchelchenkette überbrücken können. Allerdings ist bei allen diesen bekannten aktiven Hörgeräten das Trommelfell durch eine Knorpelplatte ersetzt, welche lediglich zur Abdichtung des Mittelohrraums nach außen dient und mit der Schallübertragung nichts mehr zu tun hat. Letztere erfolgt ausschließlich durch von außen zugeführte elektrische Impulse auf ein Mittelohrseitig hinter der Knorpelplatte angeordnetes piezoelektrisches Wandlerelement.

Konkret sind in der DE 10 2004 038 078 A1 zwei Ausführungsformen gezeigt, bei denen durch "passive" Teile des Hörgeräts jeweils ausgehend vom Wandlerelement einmal die Distanz zu einem vollständig erhaltenen Steigbügel und zum anderen die Distanz zur Steigbügelfußplatte überbrückt wird. Im ersteren Fall erfolgt eine Ankopplung an den Steigbügelkopf, im zweiten Fall an einen Kugelkopf, von welchem dann ein weiterer Teil des Hörgeräts direkt zur Steigbügelfußplatte verläuft.

In beiden Fällen wird eine form- und kraftschlüssige Ankopplung jeweils durch einen räumlich glockenartig aufgebauten Clip bewirkt. Die Verwendung einer Schlinge wie bei einer gattungsgemäßen passiven Gehörknöchelchenprothese der eingangs beschriebenen Art verbietet sich hier schon deshalb, weil ein schlingenförmiges Ankoppelelement seitlich leicht vom Steigbügelkopf oder vom Kugelkopf herunter rutschen würde. Umgekehrt wäre aber der bekannte glockenartig aufgebaute Clip wiederum nicht als Koppelelement zur mechanischen Verbindung mit dem Ambossfortsatz oder mit dem Hammergriff geeignet, weil er sich gar nicht daran anbringen ließe.

Eine aus dem oben zitierten deutschen Gebrauchsmuster DE 296 09 687 U1 der Anmelderin bekannte Gehörknöchelchenprothese zeichnet sich dadurch aus, dass sie sich durch einfaches Aufklippen auf den langen Ambossfortsatz relativ leicht implantieren lässt. Sie hält allein durch die Klemmwirkung des Clips. Weitere Befestigungsmittel oder spezielle Fixierverfahren sind überflüssig.

Dennoch könnte es im Verlauf einer Implantation zu Verkippungen der Prothese kommen, wodurch die Operation für den Chirurgen erschwert würde. Um diesem Problem zu begegnen, ist bei einem "Nachfolgemodell" dieser Prothese, das in der DE 202 12 771 U1 beschrieben ist, die Klammer mit mindestens einem ihrer Schenkel verlängert, der über die Öffnung nach außen in Form eines Bogens ragt, mit dem die Prothese am langen Ambossfortsatz des menschlichen Mittelohrs vor dem Aufschieben aufhängbar ist, wodurch der Operateur die Möglichkeit erhält, das Operationsbesteck zu wechseln und z.B. ein Häkchen zum Aufschieben der Prothese auf den langen Ambossfortsatz zu verwenden. Außerdem kann durch diese Maßnahme die Federkennlinie des Gebildes erheblich "weicher" gestaltet werden. Dies hat eine wesentlich verbesserte Handhabung der Gehörknöchelchenprothese zur Folge, weil dadurch auch größere Unterschiede in der individuellen Form und Größe der Ankoppelstelle am Gehörknöchelchen des jeweiligen Patienten leicht ausgeglichen werden können, ohne dass jeweils unterschiedliche Prothesen vorgehalten werden müssen.

Die bekannten Gehörknöchelchenprothesen nach den eingangs zitierten WO 02/069850 A1 oder der US 6,554,861 B2, die aus einem Metall mit so genanntem Memory-Effekt aufgebaut sind, lassen sich mit Hilfe des jeweils als Schlinge ausgebildeten ersten Befestigungselements ebenfalls relativ leicht an einer gewünschten Stelle eines Gehörknöchelchens fixieren. Durch Einbringung von Wärme gibt man dem Memory-Metall, beispielsweise Nitinol, den Impuls, sich zu schließen. Die Prothesen-Schlinge "erinnert" sich dann an ihren Urzustand und wird so am Ambossfortsatz befestigt. Damit lässt sich der etwas aufwändiger zu fertigende aufschiebbare Clip gemäß der DE 296 09 687 U1 oder der DE 202 12 771 U1 scheinbar elegant umgehen.

Die bekannten Schlingen-Prothesen der in WO 02/069850 A1 oder US 6,554,861 B2 beschriebenen Art haben jedoch den erheblichen Nachteil, dass sie die entsprechende Ankoppelstelle am Gehörknöchelchen, in der Regel den Ambossfortsatz oder den Hammergriff, komplett umschließen, was in der Praxis oftmals dazu führen kann, dass die auf dem umschlossenen Knochenbereich liegende Schleimhaut stranguliert wird und damit die dahinter liegenden Strukturen absterben.

Dieses Problem wird zwar durch eine Gehörknöchelchenprothese der Anmelderin gemäß der EP 0 809 982 B1 gelöst, jedoch handelt es sich hierbei wiederum um eine Prothese mit Clip als Befestigungselement.

Der vorliegenden Erfindung liegt demgegenüber nun die Aufgabe zugrunde, die bekannten Schlingen-Prothesen mit möglichst einfachen Mitteln so auszugestalten, dass einerseits möglichst viele unterschiedliche Geometrien und Außendurchmesser der individuellen Ankoppelstellen im Mittelohr des jeweiligen Patienten mit einer einzigen Gehörknöchelchenprothese abgedeckt werden können, wobei aber andererseits eine vollständige Strangulierung der Ankoppelstelle am Gehörknöchelchen vermieden und die Gefäße innerhalb der Knochenschleimhaut nicht beschädigt werden sollen bzw. diese sich nach der Befestigung der Gehörknöchelchenprothese einen neuen Weg bahnen können.

Diese komplexe Aufgabe wird erfindungsgemäß mit einer Gehörknöchelchenprothese der eingangs genannten Art auf verblüffend einfache, aber wirkungsvolle Weise dadurch gelöst, dass die Schlinge derart ausgebildet ist, dass sie nach der Ankopplung der Gehörknöchelchenprothese am umgriffenen Glied mit mindestens drei Bereichen kraftschlüssig anliegt, und dass in Umfangsrichtung um das umgriffene Glied herum mindestens drei Bereiche der Schlinge, von denen einer die spaltartige Öffnung umfassen kann, das von der Schlinge umgriffene Glied nicht berühren, sondern jeweils mit Abstand von der Oberfläche des umgriffenen Gliedes verlaufen, wobei die am umgriffenen Glied anliegenden Bereiche und die das umgriffene Glied nicht berührenden Bereiche der Schlinge sich in Umfangsrichtung um das umgriffene Glied jeweils abwechseln.

Auf diese Weise können ohne größeren technischen Aufwand und auf simple Weise die Vorteile der oben beschriebenen bekannten Gehörknöchelchenprothese gemäß der gattungsbildenden Druckschriften WO 02/069850 A1 oder US 6,554,861 B2 genutzt werden, wobei aber deren wesentliche Nachteile gegenüber den Prothesen gemäß den Druckschriften DE 296 09 687 U1, DE 202 12 771 U1 oder EP 0 809 982 B1 elegant umgangen werden. Wie bei dem in der Herstellung etwas anspruchsvolleren und aufwändigeren "aufschiebbaren Clip mit Lücken" werden durch die modifizierte Schlinge des ersten Befestigungselements der erfindungsgemäßen Gehörknöchelchenprothese die Versorgungsgefäße am Gehörknöchelchen nur in den Anlagebereichen tangiert. Die übrigen Gefäße verlaufen in den Bereichen, in denen die Schlinge nicht am Gehörknöchelchen anliegt, sodass die Nährstoffversorgung des Gehörknöchelchens, beispielsweise des langen Ambossfortsatzes und des Proc. Lenticularis, nicht gefährdet wird.

Eine besonders bevorzugte Ausführungsform der erfindungsgemäßen Gehörknöchelchenprothese zeichnet sich dadurch aus, dass die Schlinge aus flachem Bandmaterial aufgebaut ist und flächig am umgriffenen Glied der Gehörknöchelchekette anliegt, wodurch der lokal vom Befestigungselement auf den Knochen ausgeübte Druck großflächig verteilt und damit ganz erheblich reduziert werden kann. Dadurch kann die Ausbildung von Einschnürungen und damit das potentielle Auftreten von lokalen Nekrosen verhindert werden.

Alternativ kann die Schlinge aber auch aus Drahtmaterial aufgebaut sein, was die Herstellung vereinfacht, aber eben die oben beschriebenen Nachteile mit sich führen kann.

Letztere können bei Weiterbildungen dieser einfachen Ausführungsform dadurch verhindert oder zumindest entscheidend abgemildert werden, dass das Drahtmaterial einen rechteckigen Querschnitt aufweist und dass die Schlinge mit einer Rechteckfläche am umgriffenen Glied der Gehörknöchelchenkette anliegt. Hierdurch wird ebenso wie bei der Schlinge aus Bandmaterial die Flächenpressung und damit der auf den Knochen ausgeübte Druck verteilt und lokal reduziert.

Ganz besonders bevorzugt sind Ausführungsformen der Erfindung, bei denen die Anlagepunkte des Befestigungselements am Gehörknöchelchen so verteilt sind, dass eine verzerrungsfreie Signalübertragung gewährleistet wird. Dies lässt sich dadurch erreichen, dass die Bereiche, mit denen die Schlinge am umgriffenen Glied der Gehörknöchelchenkette kraftschlüssig anliegt, symmetrisch um den Umfang des umgriffenen Gliedes verteilt angeordnet sind.

In der Regel wird bei der erfindungsgemäßen Gehörknöchelchenprothese die Schlinge am einen Ende eines länglichen Schafts angeordnet sein, der die Schlinge mit dem anderen Ende der Gehörknöchelchenprothese verbindet, wie dies an sich aus dem Stand der Technik wohlbekannt ist.

Bei einer geometrisch besonders bevorzugten Weiterbildung dieser Klasse von Ausführungsformen ist ein Bereich, mit dem die Schlinge am umgriffenen Glied der Gehörknöchelchenkette kraftschlüssig anliegt, in unmittelbarer Fortsetzung des Schaftes an dessen schlingenseitigem Ende angeordnet. Dadurch wird in Richtung der Signalübertragung durch die Gehörknöchelchenprothese ein drehmomentfreier Krafteintrag direkt an der Stelle der mechanischen Ankopplung des ersten Befestigungselements am Gehörknöchelchen erreicht.

Alternativ oder zusätzlich kann bei Weiterbildungen der obigen Ausführungsformen vorgesehen sein, dass ein Bereich, mit dem die Schlinge am umgriffenen Glied der Gehörknöchelchenkette kraftschlüssig anliegt, der unmittelbaren Fortsetzung des Schaftes an seinem schlingenseitigen Ende bezüglich des umgriffenen Gliedes diametral gegenüber liegend angeordnet ist, wodurch wiederum ein günstiger Krafteintrag bei der Signalübertragung erreicht wird.

Andere Weiterbildungen der oben beschriebenen Klasse von Ausführungsformen zeichnen sich dadurch aus, dass ein Bereich, der die spaltartige Öffnung der Schlinge umfassen kann und das von der Schlinge umgriffene Glied der Gehörknöchelchenkette nicht berührt, in unmittelbarer Fortsetzung des Schaftes an dessen schlingenseitigem Ende angeordnet ist.

Nachdem die erfindungsgemäße Prothese operativ im Mittelohr platziert wurde und das Trommelfell wieder verschlossen ist, beginnt die so genannte Einheilphase. In dieser Zeit bilden sich Narben, und diese verursachen unvorhersehbar Kräfte, welche dazu führen können, die Prothese aus ihrer lokalen Position zu verschieben. Aus diesem Grund ist es sehr hilfreich, wenn sich die Prothese postoperativ selbstständig einer geänderten Position im Mittelohr angleichen kann. Da zudem die anatomischen Gegebenheiten des Ohrs, wie beispielsweise die Lage, die Form und die Größe des Steigbügels, des Ambosses, des Hammers und des Trommelfells individuell variieren, ist es generell sehr vorteilhaft, wenn Gehörknöchelchenprothesen nicht starr ausgebildet sind, sondern eine gewisse Flexibilität oder Variabilität aufweisen. Um diese Flexibilität bzw. Variabilität zu erreichen, sind verschiedene Befestigungs- und Ankopplungsvorrichtungen für Gehörknöchelchen, die elastische Teile und/oder Gelenke aufweisen, bekannt. Eine solche gelenkige Verbindung zwischen einem an der Steigbügelfußplatte montierbaren Befestigungselement und einem länglichen Schaft der Gehörknöchelchenprothese ist an sich in der EP 1 181 907 B1 beschrieben und wird von der Anmelderin unter dem Markennamen "Ball-Joint-Prothese" angeboten. Bei einer besonders bevorzugten Ausführungsform der erfindungsgemäßen Gehörknöchelchenprothese ist daher am oder im länglichen Schaft mindestens ein Kugelgelenk vorgesehen.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager "dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer wachstumsfördernden und/oder einer antibakteriell wirkenden Beschichtung überzogen.

Die erfindungsgemäße Gehörknöchelchenprothese selbst oder Teile davon können aus Titan und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Prothese oder Teile davon, insbesondere die Schlinge, aus einem Material mit Formgedächtnis (=memory effect) oder superelastischen Eigenschaften, vorzugsweise aus Nitinol hergestellt sind, was per se beispielsweise aus den eingangs zitierten Druckschriften WO 02/069850 A1 oder US 6,554,861 B2 bekannt ist.

Möglich sind aber auch Ausführungsformen der Erfindung, bei denen die Prothese oder Teile davon aus biokompatiblen Kunststoffen, insbesondere Silikon, Polytetrafluorethylen (PTFE) oder Faserverbundwerkstoffen hergestellt ist. Mit diesen Materialien können postoperative Abstoßungsreaktionen in den meisten Fällen ebenfalls verhindert werden.

Besonders bevorzugt ist eine Ausführungsform der erfindungsgemäßen Vorrichtung, bei der die Massenverteilung der einzelnen Teile der Prothese in Abhängigkeit von einem gewünschten, vorgebbaren Frequenzgang der Schallleitung im Mittelohr berechnet ist. Damit lässt sich ohne großen zusätzlichen technischen Aufwand gewissermaßen ein Tuning der Schallfortpflanzungseigenschaften mittels einer individuellen ausgestalteten Gehörknöchelchenprothese erreichen.

Ein solcher Tuning Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist.

Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein. Bei vielen Ausführungsformen kann beispielsweise die Prothese einerseits am Ambossfortsatz und andererseits am Steigbügel befestigt oder direkt ins Innenohr getaucht werden. Bei wieder anderen Ausbildungen der Erfindung ist die Prothese einerseits am Hammergriff und andererseits am Amboss oder am Steigbügel befestigt oder wird direkt ins Innenohr getaucht. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird. Weitere besonders bevorzugte Ausführungsformen der erfindungsgemäßen Vorrichtung zeichnen sich dadurch aus, dass die Gehörknöchelchenprothese mittels Perforation der Steigbügelfußplatte (=Stapedektomie bzw. Stapedotomie) und/oder mittels Eröffnung der menschlichen Hörschnecke (=Cochleotomie) einenends direkt an das Innenohr angekoppelt ist, insbesondere über einen Kolben.

Bei alternativen Ausführungsformen der erfindungsgemäßen Gehörknöchelchenprothese kann an dem an dem der Schlinge entgegengesetzten anderen Ende der Prothese ein insbesondere ebenfalls als Schlinge, als geschlossene Glocke, als einfach oder mehrfach geschlitzte Glocke oder als Clip ausgebildetes zweites Befestigungselement zur mechanischen Verbindung mit einem weiteren Glied der Gehörknöchelchenkette angeordnet sein.

Bei weiteren, besonders einfachen Ausführungsformen ist an dem der Schlinge entgegengesetzten anderen Ende der Prothese ein insbesondere als Stempel oder einfacher Draht ausgebildetes zweites Befestigungselement zur mechanischen Verbindung mit der Steigbügelfußplatte ohne Perforation angeordnet.

Eine weitere Ausführungsform der Erfindung zeichnet sich schließlich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Prothese mit der Außenwelt aufgrund der oben beschriebenen Beschichtung wiederum keine Probleme durch einen erhöhten Bakterieneintrag in den Bereich des Mittelohres verursacht, wenn die Beschichtung entsprechend antibakteriell ausgestaltet ist.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1: eine schematische Darstellung einer ersten Ausführungsform der erfindungsgemäßen Gehörknöchelchenprothese mit Dreipunkt-Anlage der Schlinge am Gehörknöchelchen und kraftschlüssiger Anlage der Schlinge am länglichen Schaft;
- Fig. 2: eine weitere Ausführungsform mit Dreipunkt-Anlage der Schlinge am Gehörknöchelchen, jedoch keiner kraftschlüssiger Anlage am Schaft; und
- Fig. 3: eine schematische Darstellung einer dritten Ausführungsform, mit Vierpunkt-Anlage der Schlinge am Gehörknöchelchen und kraftschlüssiger Anlage der Schlinge am länglichen Schaft.

Bei sämtlichen Figuren der Zeichnung ist das umgriffenen Glied der Gehörknöchelchen mit der Bezugsziffer 1 bezeichnet und schematisch in einem Schnitt dargestellt. Außerdem weisen alle gezeigten Gehörknöchelchenprothesen 10; 20; 30 jeweils an ihrem einen Ende ein erstes Befestigungselement in Form einer Schlinge 11; 21; 31 und an ihrem entgegengesetzten Ende einen Kolben 12; 22; 32 auf. Die Schlinge 11; 21; 31 ist jeweils durch einen länglichen Schaft 13; 23; 33 mit dem Kolben 12; 22; 32 verbunden. Wie oben beschrieben, können andere, nicht in der Zeichnung dargestellte Ausführungsformen der erfindungsgemäßen Gehörknöchelchenprothese an ihrem der Schlinge entgegengesetzten Ende statt eines Kolbens auch ein anderes Befestigungselement aufweisen, beispielsweise eine weitere Schlinge, einen Clip, eine Glocke oder Ähnliches.

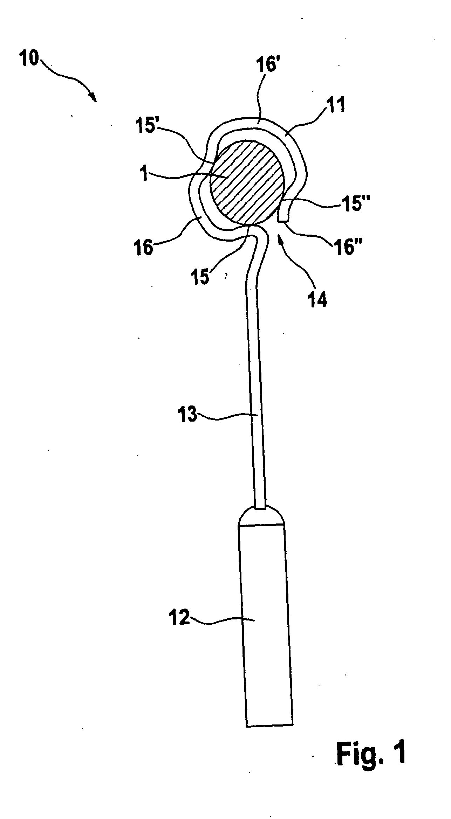

Fig. 1 stellt schematisch eine Gehörknöchelchenprothese 10 dar, bei der die Schlinge 11 nach der Ankopplung der Prothese 10 am umgriffenen Glied 1 mit genau drei Bereichen 15, 15', 15" kraftschlüssig anliegt, wobei in Umfangsrichtung um das umgriffene Glied 1 herum weitere drei Bereiche 16, 16', 16" der Schlinge 11, von denen einer eine spaltartige Öffnung 14 umfasst, das von der Schlinge 11 umgriffene Glied 1 nicht berühren, sondern jeweils mit Abstand von der Oberfläche des umgriffenen Gliedes 1 verlaufen. Die punktartige Anlage (bzw. eine klein gehaltene Anlagefläche) der Schlinge 11 in den drei Bereichen 15, 15', 15" sorgen für eine sichere Verbindung. Der erste Anlagepunkt 15 liegt bei der 6-Uhr-Position bezüglich des Schaftes 13, der zweite Anlagepunkt 15' bei der 10-Uhr-Position und der dritte Anlagepunkt 15" bei der 16-Uhr-Position. Ein besonderer Vorteil dieser symmetrischen Anordnung ist ein Krafteintrag bei der 6-Uhr-Position in Richtung der Signalübertragung, wobei kein Drehmoment auftritt.

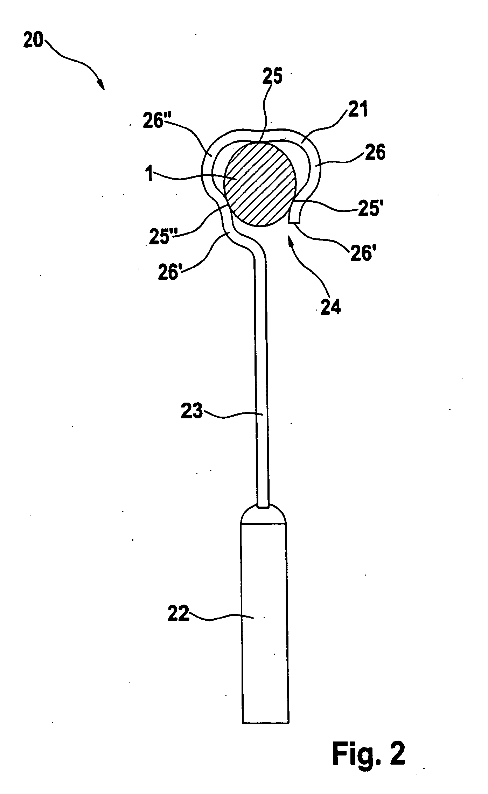

Die in Fig. 2 gezeigte Gehörknöchelchenprothese 20 weist ebenfalls eine symmetrische Drei-Punkt-Anlage in den drei Bereichen 25, 25', 25" auf, während weitere drei Bereiche 26, 26', 26" das von der Schlinge 21 umgriffene Glied 1 der Gehörknöchelchenkette nicht berühren. Allerdings ist hier der Bereich 26', der die spaltartige Öffnung 24 der Schlinge 21 umfasst, in unmittelbarer Fortsetzung des Schaftes 23 an dessen schlingenseitigem Ende angeordnet

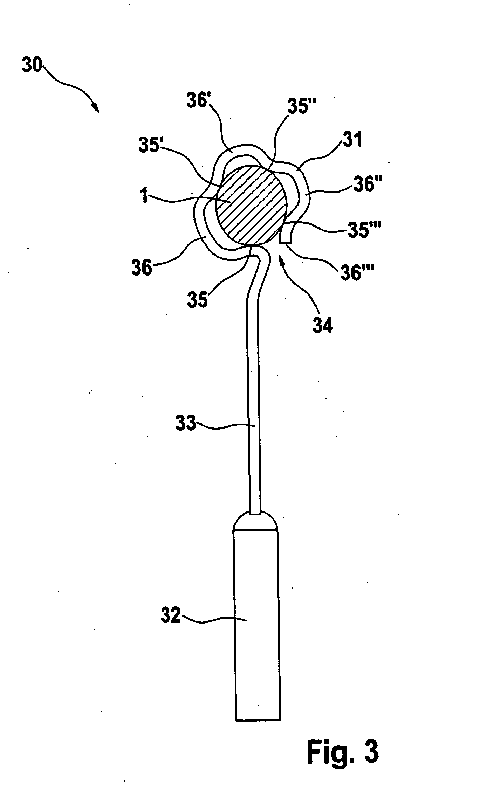

Die Prothese 30 in Fig. 3 schließlich weist eine symmetrische Vier-Punkt-Anlage in den vier Bereichen 35, 35', 35", 35"' auf, während weitere vier Bereiche 36, 36', 36", 36'" das von der Schlinge 31 umgriffene Glied 1 der Gehörknöchelchenkette nicht berühren. Der erste Anlagepunkt liegt hier bezüglich des Schaftes 33 wie bei der Ausführungsform nach Fig. 1 an der 6-Uhr-Position, der zweite bei der 10-Uhr-Position, der dritte bei der 13-Uhr-Position und der vierte bei der 16-Uhr-Position. Vorteilhaft bei dieser Ausführungsform ist wiederum ein drehmomentfreier Krafteintrag bei der 6-Uhr-Position in Richtung der Signalübertragung. Außerdem entsteht keine zu große Lücke zwischen den Anlagepunkten, welche bei kleinem Ambossfortsatz zu ungenügender Fixierung der Prothese führen könnte.

Die erfindungsgemäßen Gehörknöchelchenprothesen können sich unter anderem in ihrer Länge und auch in der Stärke des verwendeten Materials unterscheiden.

Um eine gewisse Gelenkigkeit zu erhalten, können in der Zeichnung ebenfalls nicht dargestellte Ausführungsformen der Erfindung eine Gelenkstelle oder eine Vielzahl von aneinander angreifenden Gelenkstellen aufweisen, die in der Regel am bzw. im länglichen Schaft 13; 23; 33 angeordnet sein werden oder diesen ersetzen.

Zur zusätzlichen Verbesserung der Hörqualität kann bei weiteren in der Zeichnung nicht dargestellten Ausführungsformen eine Masse am länglichen Schaft 13; 23; 33 angebracht sein, die einem Feintuning der akustischen Eigenschaften der Gehörknöchelchenprothese durch gezielte Verschiebung der Resonanzfrequenz auf einen gewünschten Wert dient.

Außerdem kann die äußere Oberfläche der Gehörknöchelchenprothese 10; 20; 30 mit einer biologisch aktiven, je nach Bedarf wachstumshemmenden oder wachstumsfördernden Beschichtung versehen sein. Eine wachstumshemmende Beschichtung ist insbesondere im Durchtrittsbereich des Kolbens 12; 22; 32 durch die Öffnung in der Steigbügelfußplatte von besonderer Wichtigkeit, da hier die Prothese im Innenohr sitzt und schwingen soll, sodass ein Anwachsen an dieser Stelle auf jeden Fall verhindert werden muss. Die wachstumshemmende Beschichtung wirkt hier also wie eine Trennschicht. Die Beschichtung kann auch keimtötende, insbesondere antibakterielle Wirkungen haben und nach Implantation der Prothese 10; 20; 30 im Mittelohr selbsttätig über einen längeren Zeitraum kontinuierlich Substanzen, insbesondere Antibiotika an ihre Umgebung abgeben.

## Patentansprüche

1. Passive Gehörknöchelchenprothese (10; 20; 30), die mindestens ein Glied der menschlichen Gehörknöchelchenkette ersetzt oder überbrückt, wobei die Gehörknöchelchenprothese (10; 20; 30) zumindest an einem Ende ein erstes Befestigungselement zur mechanischen Verbindung mit einem Glied (1) der Gehörknöchelchenkette, nämlich mit dem Ambossfortsatz oder mit dem Hammergriff, aufweist, das in Form einer das Glied (1) umgebenden, durch eine spaltartige Öffnung (14; 24; 34) nach außen hin teilweise offenen Schlinge (11; 21; 31) gestaltet ist, die das Glied (1) der Gehörknöchelchenkette, mit dem die mechanische Verbindung hergestellt werden soll, kraftschlüssig umgreift, und wobei die spaltartige Öffnung (14; 24; 34) der Schlinge (11; 21; 31) erheblich kleiner ist als der Durchmesser des von der Schlinge umgriffenen Gliedes (1), **dadurch gekennzeichnet, dass** die Schlinge (11; 21; 31) derart ausgebildet ist, dass sie nach der Ankopplung der Gehörknöchelchenprothese (10; 20; 30) am umgriffenen Glied (1) mit mindestens drei Bereichen (15, 15', 15"; 25, 25', 25"; 35, 35', 35", 35"') kraftschlüssig anliegt, und dass in Umfangsrichtung um das umgriffene Glied (1) herum mindestens drei Bereiche (16, 16', 16"; 26, 26', 26"; 36, 36', 36", 36"') der Schlinge (11; 21; 31), von denen einer die spaltartige Öffnung (14; 24; 34) umfassen kann, das von der Schlinge (11; 21; 31) umgriffene Glied (1) nicht berühren, sondern jeweils mit Abstand von der Oberfläche des umgriffenen Gliedes (1) verlaufen, wobei die am umgriffenen Glied (1) anliegenden Bereiche (15, 15', 15"; 25, 25', 25"; 35, 35', 35", 35"') und die das umgriffene Glied (1) nicht berührenden Bereiche (16, 16', 16"; 26, 26', 26"; 36, 36', 36", 36"') der Schlinge (11; 21; 31) sich in Umfangsrichtung um das umgriffene Glied (1) jeweils abwechseln.

2. Gehörknöchelchenprothese nach Anspruch 1, **dadurch gekennzeichnet, dass** die Schlinge (11; 21; 31) aus flachem Bandmaterial aufgebaut ist und flächig am umgriffenen Glied (1) der Gehörknöchelchenkette anliegt.

3. Gehörknöchelchenprothese nach Anspruch 1, **dadurch gekennzeichnet, dass** die Schlinge (11; 21; 31) aus Drahtmaterial aufgebaut ist.

4. Gehörknöchelchenprothese nach Anspruch 3, **dadurch gekennzeichnet, dass** das Drahtmaterial einen rechteckigen Querschnitt aufweist, und dass die Schlinge (11; 21; 31) mit einer Rechteckfläche am umgriffenen Glied (1) der Gehörknöchelchenkette anliegt.

5. Gehörknöchelchenprothese nach einem der vorhergehenden Ansprüche, **dadurch gekennzeichnet, dass** die Bereiche (15, 15', 15"; 25, 25', 25"; 35, 35', 35"), mit denen die Schlinge (11; 21; 31) am umgriffenen Glied (1) der Gehörknöchelchenkette kraftschlüssig anliegt, symmetrisch um den Umfang des umgriffenen Gliedes (1) verteilt angeordnet sind.

6. Gehörknöchelchenprothese nach einem der vorhergehenden Ansprüche, **dadurch gekennzeichnet, dass** die Schlinge (11; 21; 31) am einen Ende eines länglichen Schafts (13; 23; 33) angeordnet ist, der die Schlinge (11; 21; 31) mit dem anderen Ende der Gehörknöchelchenprothese (10; 20; 30) verbindet.

7. Gehörknöchelchenprothese nach Anspruch 6, **dadurch gekennzeichnet, dass** ein Bereich (15; 35), mit dem die Schlinge (11; 31) am umgriffenen Glied (1) der Gehörknöchelchenkette kraftschlüssig anliegt, in unmittelbarer Fortsetzung des Schaftes (13; 33) an dessen schlingenseitigem Ende angeordnet ist.

8. Gehörknöchelchenprothese nach Anspruch 6 oder 7, **dadurch gekennzeichnet, dass** ein Bereich (25; 35"), mit dem die Schlinge (21; 31) am umgriffenen Glied (1) der Gehörknöchelchenkette kraftschlüssig anliegt, der unmittelbaren Fortsetzung des Schaftes (23; 33) an seinem schlingenseitigen Ende bezüglich des umgriffenen Gliedes (1) diametral gegenüber liegend angeordnet ist.

9. Gehörknöchelchenprothese nach Anspruch 6 oder 8, **dadurch gekennzeichnet, dass** ein Bereich (26'), der die spaltartige Öffnung (24) der Schlinge (21) umfassen kann und das von der Schlinge (21) umgriffene Glied (1) der Gehörknöchelchenkette nicht berührt, in unmittelbarer Fortsetzung des Schaftes (23) an dessen schlingenseitigem Ende angeordnet ist.

10. Gehörknöchelchenprothese nach einem der Ansprüche 6 bis 9, **dadurch gekennzeichnet, dass** die Gehörknöchelchenprothese (10; 20; 30) mittels Perforation der Steigbügelfußplatte und/oder mittels Eröffnung der menschlichen Hörschnecke am anderen Ende des länglichen Schafts (13; 23; 33) direkt an das Innenohr angekoppelt ist, insbesondere über einen Kolben (12; 22; 32).

11. Gehörknöchelchenprothese nach einem der Ansprüche 6 bis 9, **dadurch gekennzeichnet, dass** an dem der Schlinge (11; 21; 31) entgegengesetzten anderen Ende der Prothese ein insbesondere ebenfalls als Schlinge, als geschlossene Glocke, als einfach oder mehrfach geschlitzte Glocke oder als Clip ausgebildetes zweites Befestigungselement zur mechanischen Verbindung mit einem weiteren Glied der Gehörknöchelchenkette angeordnet ist.

12. Gehörknöchelchenprothese nach einem der Ansprüche 6 bis 9, **dadurch gekennzeichnet, dass** an dem der Schlinge (11; 21; 31) entgegengesetzten anderen Ende der Prothese ein insbesondere als Stempel oder einfacher Draht ausgebildetes zweites Befestigungselement zur mechanischen Verbindung mit der Steigbügelfußplatte ohne Perforation angeordnet ist.

13. Gehörknöchelchenprothese nach einem der Ansprüche 6 bis 12, **dadurch gekennzeichnet, dass** am oder im länglichen Schaft mindestens ein Kugelgelenk vorgesehen ist.

14. Gehörknöchelchenprothese nach einem der vorhergehenden Ansprüche, **dadurch gekennzeichnet, dass** die Prothese (10; 20; 30) oder Teile davon, insbesondere die Schlinge (11; 21; 31), aus einem Material mit Formgedächtnis, vorzugsweise aus Nitinol hergestellt ist.

15. Gehörknöchelchenprothese nach einem der vorhergehenden Ansprüche, **dadurch gekennzeichnet, dass** die Prothese (10; 20; 30) oder Teile davon aus Titan und/oder aus Stahl und/oder aus Tantal und/oder aus einer Legierung der genannten Metalle hergestellt ist.

16. Gehörknöchelchenprothese nach einem der vorhergehenden Ansprüche, **dadurch gekennzeichnet, dass** die Prothese (10; 20; 30) oder Teile davon aus biokompatiblen Kunststoffen, insbesondere Silikon, Polytetrafluorethylen (PTFE) oder Faserverbundwerkstoffen hergestellt ist.

17. Gehörknöchelchenprothese nach einem der vorhergehenden Ansprüche, **dadurch gekennzeichnet, dass** zumindest abschnittsweise eine biologisch aktive Beschichtung, insbesondere eine wachstumshemmende und/oder eine wachstumsfördernde und /oder eine antibakteriell wirkende Beschichtung, vorgesehen ist.

18. Gehörknöchelchenprothese nach einem der vorhergehenden Ansprüche, **dadurch gekennzeichnet, dass** die Massenverteilung der einzelnen Teile der Prothese (10; 20; 30) in Abhängigkeit von einem gewünschten, vorgebbaren Frequenzgang der Schallleitung im Mittelohr berechnet ist.

19. Gehörknöchelchenprothese nach einem der vorhergehenden Ansprüche, **dadurch gekennzeichnet, dass** mindestens eine zusätzliche Masse in Abhängigkeit von einem gewünschten, vorgebbaren Frequenzgang der Schallleitung im Mittelohr an einem Teil der Gehörknöchelchenkette bzw. der Prothese (10; 20; 30) befestigt ist.

## Claims

1. Passive auditory ossicle prosthesis (10; 20; 30) which replaces or bridges at least one element of the human ossicular chain, wherein the auditory ossicle prosthesis (10; 20; 30) is provided at least at one end with a first fixing element for mechanical connection to an element (1) of the ossicular chain, namely to the process of incus or to the manubrium of malleus, designed in the form of a loop (11; 21; 31) surrounding the element (1), partially open towards the outside by means of a slit-like opening (14; 24; 34) and engaging non-positively around the element (1) of the ossicular chain to which the mechanical connection is to be established, the slit-like opening (14; 24; 34) of the loop (11; 21; 31) being considerably smaller than the diameter of the element (1) engaged by the loop, **characterised in that** the loop (11; 21; 31) is designed in such a manner that, after the coupling of the auditory ossicle prosthesis (10; 20; 30), it bears non-positively against the engaged element (1) by means of at least three areas (15, 15', 15"; 25, 25', 25"; 35, 35', 35", 35"') and that, in the circumferential direction around the engaged element (1), at least three areas (16, 16', 16"; 26, 26', 26"; 36, 36', 36", 36"') of the loop (11; 21; 31), one of which may include the slit-like opening (14; 24; 34), do not contact the element (1) engaged by the loop (11; 21; 31) but extend at a distance from the surface of the engaged element (1), the areas (15, 15', 15"; 25, 25', 25"; 35, 35', 35", 35"') of the loop (11; 21; 31) bearing against the engaged element (1) and the areas (16, 16', 16"; 26, 26', 26"; 36, 36', 36", 36"') thereof not contacting the engaged element (1) alternating in the circumferential direction around the engaged element (1).

2. Auditory ossicle prosthesis according to claim 1, **characterised in that** the loop (11; 21; 31) is made of flat strip material and bears flat against the engaged element (1) of the ossicular chain.

3. Auditory ossicle prosthesis according to claim 1, **characterised in that** the loop (11; 21; 31) is made of wire material.

4. Auditory ossicle prosthesis according to claim 3, **characterised in that** the wire material has a rectangular cross section and that the loop (11; 21; 31) bears against the engaged element (1) of the ossicular chain by means of a rectangular surface.

5. Auditory ossicle prosthesis according to one of the preceding claims, **characterised in that** the areas (15, 15', 15"; 25, 25', 25"; 35, 35', 35") by means of which the loop (11; 21; 31) bears non-positively against the engaged element (1) of the ossicular chain are arranged in such a manner that they are distributed symmetrically around the circumference of the engaged element (1).

6. Auditory ossicle prosthesis according to one of the preceding claims, **characterised in that** the loop (11; 21; 31) is arranged at one end of an elongated shaft (13; 23; 33) connecting the loop (11; 21; 31) to the other end of the auditory ossicle prosthesis (10; 20; 30).

7. Auditory ossicle prosthesis according to claim 6, **characterised in that** an area (15; 35) by means of which the loop (11; 31) bears non-positively against the engaged element (1) of the ossicular chain is arranged at the loop end of the shaft (13; 33) as a direct continuation thereof.

8. Auditory ossicle prosthesis according to claim 6 or claim 7, **characterised in that** an area (25; 35") by means of which the loop (21; 31) bears non-positively against the engaged element (1) of the ossicular chain is arranged diametrically opposite the direct continuation of the shaft (23; 33) at the loop end thereof in relation to the engaged element (1).

9. Auditory ossicle prosthesis according to claim 6 or claim 8, **characterised in that** an area (26') which may include the slit-like opening (24) of the loop (21) and does not contact the element (1) of the ossicular chain engaged by the loop (21) is arranged at the loop end of the shaft (23) as a direct continuation thereof.

10. Auditory ossicle prosthesis according to one of claims 6 to 9, **characterised in that** the auditory ossicle prosthesis (10; 20; 30) is coupled directly to the inner ear at the other end of the elongated shaft (13; 23; 33), in particular via a piston (12; 22; 32), by means of perforation of the base of stapes and/or opening of the human cochlea.

11. Auditory ossicle prosthesis according to one of claims 6 to 9, **characterised in that** a second fixing element for mechanical connection to another element of the ossicular chain, in particular also designed as a loop, a closed bell, a bell provided with one or more slits, or a clip, is arranged at the other end of the prosthesis opposite the loop (11; 21; 31).

12. Auditory ossicle prosthesis according to one of claims 6 to 9, **characterised in that** a second fixing element for mechanical connection to the base of stapes without perforation, in particular designed as a stamp or a single wire, is arranged at the other end of the prosthesis opposite the loop (11; 21; 31).

13. Auditory ossicle prosthesis according to one of claims 6 to 12, **characterised in that** at least one ball joint is provided on or in the elongated shaft.

14. Auditory ossicle prosthesis according to one of the preceding claims, **characterised in that** the prosthesis (10; 20; 30) or parts thereof, in particular the loop (11; 21; 31), is/are made of a memory-effect material, preferably nitinol.

15. Auditory ossicle prosthesis according to one of the preceding claims, **characterised in that** the prosthesis (10; 20; 30) or parts thereof is/are made of titanium and/or steel and/or tantalum and/or an alloy of the aforesaid metals.

16. Auditory ossicle prosthesis according to one of the preceding claims, **characterised in that** the prosthesis (10; 20; 30) or parts thereof is/are made of biocompatible plastics, in particular, silicone, polytetrafluoroethylene (PTFE) or fibre composites.

17. Auditory ossicle prosthesis according to one of the preceding claims, **characterised in that** a biologically active coating, in particular a growth-inhibiting and/or growth-promoting and/or anti-bacterial coating, is provided at least in some areas.

18. Auditory ossicle prosthesis according to one of the preceding claims, **characterised in that** the weight distribution of the individual parts of the prosthesis (10; 20; 30) is calculated as a function of a desired predetermined frequency response for sound conduction in the middle ear.

19. Auditory ossicle prosthesis according to one of the preceding claims, **characterised in that** at least one additional mass is fixed to part of the ossicular chain or prosthesis (10; 20; 30) as a function of a desired predetermined frequency response for sound conduction in the middle ear.

## Revendications

1. Prothèse passive (10 ; 20 ; 30) des osselets de l'oreille, qui remplace ou raccorde au moins un élément de la chaîne d'osselets de l'oreille humaine, dans laquelle la prothèse (10 ; 20 ; 30) des osselets de l'oreille présente, au moins à une extrémité, un premier élément de fixation pour un raccordement mécanique avec un élément (1) de la chaîne des osselets de l'oreille, à savoir avec le prolongement de l'enclume ou avec le manche du marteau, qui se présente sous la forme d'une boucle (11 ; 21 ; 31) entourant l'élément (1) et partiellement ouverte vers l'extérieur à travers une ouverture de type fente (14 ; 24 ; 34), laquelle boucle enveloppe par adhérence l'élément (1) de la chaîne des osselets de l'oreille avec lequel on souhaite établir la liaison mécanique, et dans laquelle l'ouverture de type fente (14 ; 24 ; 34) de la boucle (11 ; 21 ; 31) est considérablement plus petite que le diamètre de l'élément (1) enveloppé par la boucle, **caractérisée en ce que** la boucle (11 ; 21 ; 31) est conformée de manière que, après accouplement de la prothèse (10 ; 20 ; 30) des osselets de l'oreille, elle s'applique par adhérence sur l'élément enveloppé (1) via au moins trois régions (15, 15', 15" ; 25, 25', 25" ; 35, 35', 35", 35"'), et que, dans la direction périphérique autour de l'élément enveloppé (1), au moins trois régions (16, 16', 16" ; 26, 26', 26" ; 36, 36', 36", 36"') de la boucle (11 ; 21 ; 31), dont l'une peut comprendre l'ouverture de type fente (14 ; 24 ; 34), ne touchent pas l'élément (1) enveloppé par la boucle (11 ; 21 ; 31), mais s'étendent respectivement à une certaine distance de la surface de l'élément enveloppé (1), les régions (15, 15', 15" ; 25, 25', 25" ; 35, 35', 35" , 35"') de la boucle (11 ; 21 ; 31) s'appliquant sur l'élément enveloppé (1) et celles (16, 16', 16" ; 26, 26', 26" ; 36, 36', 36", 36"') ne touchant pas l'élément enveloppé (1) alternant respectivement autour de l'élément enveloppé (1) dans la direction périphérique.

2. Prothèse des osselets de l'oreille selon la revendication 1, **caractérisée en ce que** la boucle (11 ; 21 ; 31) est constituée d'un matériau plat en bande et s'applique à plat sur l'élément enveloppé (1) de la chaîne des osselets de l'oreille.

3. Prothèse des osselets de l'oreille selon la revendication 1, **caractérisée en ce que** la boucle (11 ; 21 ; 31) est constituée d'un matériau en fil.

4. Prothèse des osselets de l'oreille selon la revendication 3, **caractérisée en ce que** le matériau en fil présente une section transversale rectangulaire et **en ce que** la boucle (11 ; 21 ; 31) s'applique par une surface rectangulaire sur l'élément enveloppé (1) de la chaîne des osselets de l'oreille.

5. Prothèse des osselets de l'oreille selon l'une quelconque des revendications précédentes, **caractérisée en ce que** les régions (15, 15', 15" ; 25, 25', 25" ; 35, 35', 35"), par lesquelles la boucle (11 ; 21 ; 31) s'applique par adhérence sur l'élément enveloppé (1) de la chaîne des osselets de l'oreille, sont distribuées symétriquement autour de la circonférence de l'élément enveloppé (1).

6. Prothèse des osselets de l'oreille selon l'une quelconque des revendications précédentes, **caractérisée en ce que** la boucle (11 ; 21 ; 31) est aménagée à une extrémité d'une tige allongée (13 ; 23 ; 33), qui raccorde la boucle (11 ; 21 ; 31) à l'autre extrémité de la prothèse (10 ; 20 ; 30) des osselets de l'oreille.

7. Prothèse des osselets de l'oreille selon la revendication 6, **caractérisée en ce qu'**une région (15 ; 35), par laquelle la boucle (11 ; 31) s'applique par adhérence sur l'élément enveloppé (1) de la chaîne des osselets de l'oreille, est aménagée dans le prolongement direct de la tige (13 ; 33) à son extrémité du côté boucle.

8. Prothèse des osselets de l'oreille selon la revendication 6 ou 7, **caractérisée en ce qu'**une région (25 ; 35"), par laquelle la boucle (21 ; 31) s'applique par adhérence sur l'élément enveloppé (1) de la chaîne des osselets de l'oreille, est aménagée diamétralement à l'opposé par rapport au prolongement direct de la tige (23 ; 33), à son extrémité du côté boucle, par rapport à l'élément enveloppé (1).

9. Prothèse des osselets de l'oreille selon la revendication 6 ou 8, **caractérisée en ce qu'**une région (26'), qui peut comprendre l'ouverture de type fente (24) de la boucle (21) et qui ne touche pas l'élément (1) de la chaîne des osselets de l'oreille enveloppé par la boucle (21), est aménagée dans le prolongement direct de la tige (23), à son extrémité du côté boucle.

10. Prothèse des osselets de l'oreille selon l'une quelconque des revendications 6 à 9, **caractérisée en ce que** la prothèse (10 ; 20 ; 30) des osselets de l'oreille est directement couplée à l'oreille interne au moyen d'une perforation de la plaque de base de l'étrier et/ou au moyen d'une incision de la cochlée humaine à l'autre extrémité de la tige allongée (13 ; 23 ; 33), en particulier via une queue (12 ; 22 ; 32).

11. Prothèse des osselets de l'oreille selon l'une quelconque des revendications 6 à 9, **caractérisée en ce qu'**un second élément de fixation est aménagé sur l'autre extrémité de la prothèse opposée à la boucle (11 ; 21 ; 31), en particulier également élaboré sous la forme d'une boucle, d'une cloche fermée, d'une cloche fendue une ou plusieurs fois ou d'une agrafe, pour un raccordement mécanique avec un autre élément de la chaîne des osselets de l'oreille.

12. Prothèse des osselets de l'oreille selon l'une quelconque des revendications 6 à 9, **caractérisée en ce qu'**un second élément de fixation est aménagé sur l'autre extrémité de la prothèse opposée à la boucle (11 ; 21 ; 31), en particulier élaboré sous la forme d'un tampon ou d'un fil unique, pour un raccordement mécanique avec la plaque de base de l'étrier sans perforation.

13. Prothèse des osselets de l'oreille selon l'une quelconque des revendications 6 à 12, **caractérisée en ce qu'**au moins une articulation à rotule est prévue sur ou dans la tige allongée.

14. Prothèse des osselets de l'oreille selon l'une quelconque des revendications précédentes, **caractérisée en ce que** la prothèse (10 ; 20 ; 30) ou une partie de celle-ci, en particulier la boucle (11 ; 21 ; 31), est fabriquée dans un matériau à mémoire de forme, de préférence, en nitinol.

15. Prothèse des osselets de l'oreille selon l'une quelconque des revendications précédentes, **caractérisée en ce que** la prothèse (10 ; 20 ; 30), ou une partie de celle-ci, est fabriquée en titane et/ou en acier et/ou en tantale et/ou en alliage des métaux cités.

16. Prothèse des osselets de l'oreille selon l'une quelconque des revendications précédentes, **caractérisée en ce que** la prothèse (10 ; 20 ; 30), ou une partie de celle-ci, est fabriquée en matériaux synthétiques biocompatibles, en particulier en silicone, en poly(tétrafluorure d'éthylène) (PTFE) ou en matériaux fibreux composites.

17. Prothèse des osselets de l'oreille selon l'une quelconque des revendications précédentes, **caractérisée en ce qu'**il est prévu un revêtement actif au plan biologique, au moins par sections, en particulier un revêtement exerçant un effet inhibant la croissance et/ou favorisant la croissance et/ou exerçant un effet antibactérien.

18. Prothèse des osselets de l'oreille selon l'une quelconque des revendications précédentes, **caractérisée en ce que** la distribution de masse des parties individuelles de la prothèse (10 ; 20 ; 30) est calculée en fonction d'une réponse harmonique prédéfinissable souhaitée de la conduction acoustique dans l'oreille moyenne.

19. Prothèse des osselets de l'oreille selon l'une quelconque des revendications précédentes, **caractérisée en ce qu'**au moins une masse supplémentaire est fixée sur une partie de la chaîne des osselets de l'oreille ou de la prothèse (10 ; 20 ; 30) en fonction d'une réponse harmonique prédéfinissable souhaitée de la conduction acoustique dans l'oreille moyenne.
